# EUROPÄISCHE PATENTANMELDUNG

(11) **EP 2 130 535 A1**
(43) Veröffentlichungstag der Anmeldung: **09.12.2009**
(21) Anmeldenummer: 09160519.6
(22) Anmeldetag: 18.05.2009
(51) Int. Cl.: A61K 31/352, A61K 31/565, A61P 5/30

(54) **Verwendung von Isoflavonen für die Herstellung eines Medikaments**

(30) Priorität: 04.06.2008 AT 8982008
(71) Anmelder: Apomedica pharmazeutische Produkte GmbH, 8010 Graz (AT)
(72) Erfinder: Böhm, Friedrich, 8010, Graz (AT)
(74) Vertreter: KLIMENT & HENHAPEL

(57) **Zusammenfassung**

Die Erfindung betrifft die Verwendung von Isoflavonen für die Herstellung eines ovulationssuppressiven antikonzeptiven Medikaments enthaltend Ethinylöstradiol mit reduzierten, Östrogen-induzierten Nebenwirkungen.

## Beschreibung

Die Erfindung betrifft die Verwendung von Isoflavonen für die Herstellung eines ovulationssuppressiven antikonzeptiven Medikaments enthaltend Ethinylöstradiol mit reduzierten, Östrogen-induzierten Nebenwirkungen.

Orale Verhütungsmittel auf der Basis von Ethinylöstradiol sind in der Empfängnisverhütung hoch wirksam, und finden weltweit breite Verwendung. Ethinylöstradiol ist dabei ein künstliches Östrogen, das sowohl im zentralen Nervensystem, als auch in Geweben des Urogenitaltraktes wirkt, wodurch die Reproduktionsfunktion inhibiert wird. Von oralen Verhütungsmitteln dieser Art wurde festgestellt, dass sie neben der effektiven Verhütung von Schwangerschaft auch andere Vorteile bereitstellen. So konnte etwa gezeigt werden, dass Frauen, die orale Verhütungsmittel einnehmen, ein geringeres Risiko einer Erkrankung an Beckenentzündung (PID), ektopischer Schwangerschaft, Gebärmutterkrebs und gutartigen Brusterkrankungen besitzen. Orale Verhütungsmittel können ebenso die Beseitigung von üblichen Menstruationsstörungen, einschließlich außerplanmäßiger Regel, prämenstrueller Spannung, übermäßigem Blutverlust und Krämpfen bewirken.

Die Verwendung bekannter, oraler Verhütungsmittel ist jedoch mitunter auch von Nebenwirkungen begleitet. Diese Nebenwirkungen bestehen etwa in einer erhöhten Wahrscheinlichkeit für venöse Thromboembolismen, ischämische Herzkrankheit, cerebrovaskuläre Erkrankungen, bis zu Nebenwirkungen wie Kopfschmerzen, Übelkeit, Brustspannen, Vaginitis, Zwischenblutungen, Ausbleiben der Regel, Schlafstörungen, oder Nebenwirkungen im Zusammenhang mit Blutdruck und der Blutgerinnung. Diese Nebenwirkungen werden zu einem großen Anteil der Östrogen-Komponente, also typischerweise Ethinylöstradiol, zugeschrieben.

Zur Erforschung und Bekämpfung dieser Nebenwirkungen wurden intensive Bestrebungen unternommen, insbesondere konnten Metabolite des Ethinylöstradiols identifiziert werden, die an der Entstehung von Nebenwirkungen großen Anteil haben. So ist bekannt, dass das Ethinylöstradiol im weiblichen Körper in unterschiedliche Varianten von Hydroxyöstrogen umgewandelt werden, wobei als Haupt-Metabolite das 16-Hydroxyöstrogen, das 4-Hydroxyöstrogen, sowie das 2-Hydroxyöstrogen zu nennen sind. Bei den vorgenannten 16- und 4-Hydroxyöstrogenen konnten Zusammenhänge mit der Entstehung von Nebenwirkungen nachgewiesen werden, da sie depurinierte Addukte an der DNA erzeugen und stark angiogenetisch und mitotisch wirken. Die angiogenetische Wirkung ist für die Reproduktion zwar von großer Bedeutung, in der Postmenopause stellt sie allerdings eine unnötige Belastung der hormonabhängigen Gewebe dar. Das letztgenannte 2-Hydroxyöstrogen wirkt antimitotisch und angiostatisch, und hat sich als gesundheitlich neutral erwiesen. Dieselben Beobachtungen treffen auch auf das 17-Betaöstradiol zu, da es in derselben Weise wie das Ethinylöstradiol metabolisiert wird. Es ist daher zu erwarten, dass eine gezielte Beeinflussung der Östrogen-Metabolisierung, bei der die Bildung von 2-Hydroxyöstrogen begünstigt, die Bildung von 16- und 4-Hydroxyöstrogen hingegen unterdrückt wird, die Entstehung von Nebenwirkungen bei der Einnahme von 17-Betaöstradiol und Ethinylöstradiol mindert.

Das Ziel der Erfindung besteht somit in der Herstellung eines ovulationssuppressiven antikonzeptiven Medikaments enthaltend Ethinylöstradiol, das über deutlich reduzierte Östrogeninduzierte Nebenwirkungen verfügt, indem die Metabolisierung des Ethinylöstradiols in günstiger Weise beeinflusst wird.

Dieses Ziel wird durch die Merkmale von Anspruch 1 erreicht. Anspruch 1 bezieht sich dabei auf die Verwendung von Isoflavonen für die Herstellung eines ovulationssuppressiven antikonzeptiven Medikaments enthaltend Ethinylöstradiol mit reduzierten, Östrogen-induzierten Nebenwirkungen. Bei den Östrogen-induzierten Nebenwirkungen des ovulationssuppressiven antikonzeptiven Medikaments handelt es sich etwa um eine erhöhte Wahrscheinlichkeit für venöse Thromboembolismen, die ischämische Herzkrankheit, cerebrovaskuläre Erkrankungen, Kopfschmerzen, Übelkeit, Brustspannen, Vaginitis, Zwischenblutungen, Ausbleiben der Regel, Schlafstörungen, sowie um Nebenwirkungen im Zusammenhang mit Blutdruck und der Blutgerinnung.

Im Sinne der Erfindung könnten Isoflavone auch für die Herstellung eines Medikaments enthaltend Ethinylöstradiol zur Behandlung von Östrogen-induzierten Beschwerden verwendet werden. Bei den Östrogen-induzierten Beschwerden kann es sich dabei um menopausale Beschwerden handeln, oder um die oben erwähnten Nebenwirkungen eines ovulationssuppressiven antikonzeptiven Medikaments.

Isoflavone gehören der Gruppe der Phytoöstrogene an, und finden sich etwa im Rotklee oder in Sojabohnen. Sie ähneln in ihrer Struktur und Wirkungsweise dem natürlichen Östrogen, üben diese Wirkung aber nicht nur durch unmittelbare Interaktion mit dem Östrogenrezeptor ER-α aus, sondern auch durch inhibitorische Effekte aufgrund ihrer Affinität zum Gegenspieler des Östrogenrezeptors ER-α, dem Östrogenrezeptor ER-β. Isoflavone binden mit deutlich höherer Affinität zum Beta-Rezeptor und können daher als SERMs (Selective estrogen receptor modulator) bezeichnet werden. SERMs zeichnen sich durch positive Effekte auf Knochen, Herz und Kreislauf aus, während Brust und Uterus nicht betroffen sind. Während Soja hinsichtlich der Isoflavone nur Genistein und Daidzein in signifikanten Konzentrationen enthält, finden sich in Rotklee zusätzlich Formononetin und Biochanin A.

Gemäß einer vorteilhaften Weiterbildung der Erfindung handelt es sich bei den verwendeten Isoflavonen insbesondere um die Aglyka Genistein, Daidzein, Glycitein, Formononetin und Biochanin A, sowie um die Glykoside Genistin, Daidzin, Glycitin, Ononin und Sissotrin, sowie um den Isoflavon-Metaboliten Equol, oder um Kombinationen hiervon.

Hinsichtlich der Erfindung wurde nun festgestellt, dass Isoflavone in der Lage sind, jene zytochromalen Enzyme zu modulieren, die das Östradiol in das 2-, 4- oder 16-Hydroxyöstrogen umwandeln, insbesondere konnte eine Aktivierung des Enzyms CYP1-A1 nachgewiesen werden. Hierzu wurde bei 8 normal zyklierenden Frauen am Tag des Eisprunges in standardisierter Weise aus dem Harn die 2-Hydroxy- und 16-Hydroxymetabolit-Konzentration gemessen. Die Frauen führten diese Messung zu Hause im Rahmen des Ovulations-Self-Monitoring durch, wobei hierzu der Harn analysiert wurde. Am Tag des Eisprunges wurde zusätzlich der Harn für die Bestimmung von 2- und 16-Hydroxyöstrogen untersucht. Dabei wurde ein EMT-Enzym-Immunoassay-Kit verwendet.

Die Messungen wurden zweimal vorgenommen, und zwar im ersten Zyklus ohne Aufnahme von Isoflavonen, und im zweiten Zyklus im Rahmen des Ovulationsmonitorings nach Einnahme von 90 mg Isoflavonen. Die Isoflavon-Einnahme begann dabei am ersten Zyklustag des zweiten Beobachtungszyklus.

Die Untersuchungen ergaben im nicht behandelten Zyklus periovulatorisch erhöhte 16-Hydroxyöstrogen-Werte mit durchschnittlich 12 ± 3.7 ng/mg Kreatinin, sowie 2-Hydroxyöstrogen-Werte von 9.77 ± 2.4 ng/mg Kreatinin. Im Behandlungszyklus hingegen lag der 16-Hydroxyöstrogen-Wert bei 4.7 ± 0.9 ng/mg Kreatinin, und der 2-Hydroxyöstrogen-Wert bei 7.6 ± 1.4 ng/mg.

Die Resultate zeigen einerseits, dass periovulatorisch eine verstärkte Hydroxylierung des Östrogens in der Position 16 erfolgt, dass aber andererseits durch eine Isoflavon-Einnahme das Gleichgewicht zugunsten des 2-Hydroxyöstrogens verschoben werden kann.

Somit ist zu erwarten, dass durch die Einnahme von Isoflavonen bei oraler Aufnahme eines Medikaments enthaltend Ethinylöstradiol die durch 16- und 4-Hydroxyöstrogen verursachten Nebenwirkungen reduziert werden. Diese Eigenschaften erweisen sich sowohl bei der Einnahme ovulationssuppressiver antikonzeptiver Medikamente enthaltend Ethinylöstradiol als vorteilhaft, als auch bei der Einnahme von Medikamenten enthaltend 17-Betaöstradiol, etwa im Zuge der Hormonersatztherapie.

## Patentansprüche

1. Verwendung von Isoflavonen für die Herstellung eines ovulationssuppressiven antikonzeptiven Medikaments enthaltend Ethinylöstradiol mit reduzierten, Östrogen-induzierten Nebenwirkungen.

2. Verwendung nach Anspruch 1, **dadurch gekennzeichnet, dass** es sich bei den Nebenwirkungen um eine erhöhte Wahrscheinlichkeit für venöse Thromboembolismen, ischämische Herzkrankheit, cerebrovaskuläre Erkrankungen, Kopfschmerzen, Übelkeit, Brustspannen, Vaginitis, Zwischenblutungen, Ausbleiben der Regel, Schlafstörungen, sowie um Nebenwirkungen im Zusammenhang mit Blutdruck und der Blutgerinnung handelt.

3. Zusammensetzung mit ovulationssuppressiver, antikonzeptiver Wirkung, **dadurch gekennzeichnet, dass** die Zusammensetzung Ethinylöstradiol und ein Isoflavon umfasst.

4. Zusammensetzung nach Anspruch 3, **dadurch gekennzeichnet, dass** es sich bei dem Isoflavon um die Aglyka Genistein, Daidzein, Glycitein, Formononetin und Biochanin A, sowie um die Glykoside Genistin, Daidzin, Glycitin, Ononin und Sissotrin, sowie um den Isoflavon-Metaboliten Equol, oder um Kombinationen hiervon handelt.
